# EUROPEAN PATENT APPLICATION

(11) **EP 1 491 526 A1**
(43) Date of publication of application: **29.12.2004**
(21) Application number: 03014712.8
(22) Date of filing: 27.06.2003
(51) Int. Cl.: C07C 273/04

(54) **Process and plant for urea production**

(71) Applicant: UREA CASALE S.A., 6900 Lugano-Besso (CH)
(72) Inventor: Romiti, Domenico, CH-6900 Lugano (CH)
(74) Representative: Zardi, Marco

(57) **Abstract**

The present invention concerns a process for urea production, with an advantageous and substantial energy recovery, of the type including a high pressure synthesis step of ammonia and carbon dioxide and a medium or low pressure urea separation step, being provided an expansion step, in a turbine, of the reaction mixture coming from said high pressure synthesis step.

## Description

### Field of application

In its most general aspect, the present invention refers to a process for urea production of the type comprising:
- an urea synthesis step through high pressure reaction between ammonia and carbon dioxide, obtaining a reaction mixture including urea and ammonium carbamate in aqueous solution;
- an urea separation step, in which the urea that is in said mixture is separated at medium or low pressure from said carbamate.

As an example, it is worth specifying that, in the present application, high pressure means values typically between 100 and 400 bar, medium pressure refers to values typically between 5 and 20 bar, even up to a maximum of 80 bar and low pressure means values typically between 1 and 5 bar.

The invention also refers to a plant for carrying out the aforementioned process, as well as to a method for transforming a pre-existing urea plant into a plant according to the invention.

As it is known, relatively to urea production, there is an ever greater need to have, on the one hand, plants equipped with greater capacity and managerial flexibility and, on the other hand, with lower investment and operating costs, in particular in terms of energy consumption.

### Prior art

In order to reduce energy consumption, a series of processes for urea production have been proposed and carried out in the field, where energy recovery is provided.

Generally, the energy recovered is fundamentally of the thermal type and is obtained with so-called double-effect technology, based upon the heat transferal between process flows.

A first example of this double-effect technology is that of operating, using the reaction heat released during the condensation of the ammonia and carbon dioxide in carbamate in the separation step, a preheating of the ammonia that is supplied in the synthesis step.

A second example of this double-effect technology is that of operating a heating on the carbamate in aqueous solution to be recycled at the section where the synthesis step takes place, again using the reaction heat which is released during the condensation of the ammonia and carbon dioxide in carbamate in the separation step.

Yet another example of this double-effect technology is that of feeding part of the ammonia and carbon dioxide vapors, obtained from the decomposition of the aqueous solution of carbamate in the separation step, to decompose, in a subsequent finishing step, remaining traces of carbamate that still are in the concentrated urea solution.

However, as one can imagine, to realize such thermal recoveries it is necessary to arrange substantial interventions on the plant, since duplications of apparatuses, with respect to a conventional plant, are in practice necessary: this consequently means a greater investment for a new plant, whereas the costs are even greater if it is necessary to transform an existing plant.

It is also worth pointing out that, in general, whilst the plant solutions may have been correctly researched, the recovery which can be obtained with the processes according to the prior art is relatively low. In practice, according to the prior art, it is recovered a low pressure vapor which can be poorly used, with the result of a very exiguous energy conservation.

### Summary of the invention

The problem underlying the present invention is that of providing a process and a plant for urea production which satisfies the requirement of carrying out a significant energy recovery, at the same time overcoming the quoted drawbacks with reference to the prior art in a simple and cost-effective manner.

This problem is solved according to the invention by a process for urea production, of the type indicated above, characterized in that it comprises an expansion step, in a turbine, of the reaction mixture coming from the high pressure synthesis step.

In the expansion step kinetic energy is produced by the turbine, with an enormously greater energy recovery efficiency with respect to that which is obtained with the prior art. Such kinetic energy can easily be used, for example, to produce electrical energy. Another very helpful use of this kinetic energy, or of the electrical energy produced by it, is that of using it to actuate pumps and compressors with many stages which are conventionally provided to take the reactants to a predetermined pressure for the synthesis step.

The advantages and characteristics of the process for urea production with energy recovery according to the present invention, as well as its relative plant and the transformation method of a pre-existing urea production plant, shall become clearer from the description of an example embodiment thereof, given hereafter with reference to the attached drawings, given for indicative and not limiting purposes.

### Brief description of the drawings

Figure 1 represents a block diagram of a plant for urea production, according to the present invention;
Figure 2 schematically represents a plant for urea production, according to the present invention.

### Detailed description of a preferred embodiment

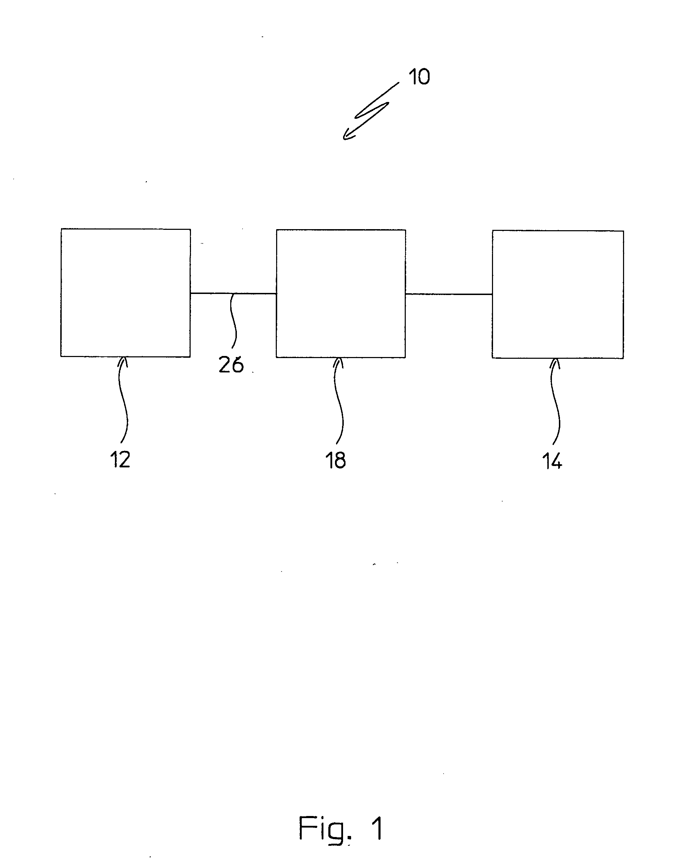

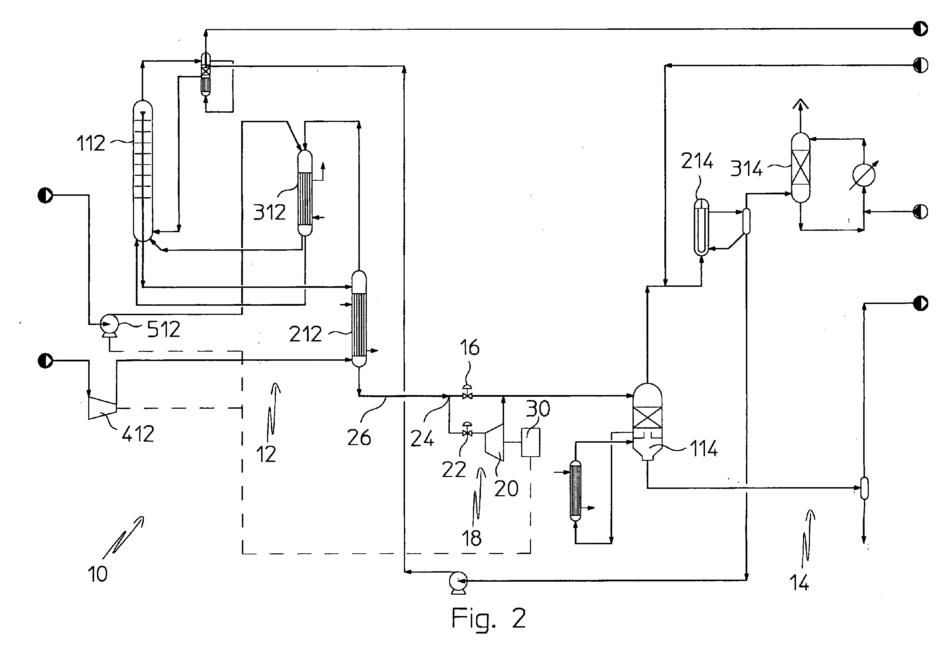

With reference to figures 1 and 2, a plant for urea production in accordance with the present invention is shown and it is globally indicated with 10.

According to the prior art, the plant 10 for urea production is equipped with a synthesis section 12 and an urea separation section 14.

In the synthesis section 12, that is conventionally provided with, for example, a compressor 412 for carbon dioxide, a pump 512 for ammonia, a reactor 112, a stripper 212 and a condenser 312, ammonia and carbon dioxide react:
in this section 12, all the apparatuses operate at high pressure.

In the subsequent urea separation section 14, that is provided with, for example, a decomposer 114, a condenser 214 and an absorber 314, it is useful to operate at a medium and/or low pressure, mainly to ease the different decomposition reactions.

According to the prior art, to reduce the pressure of the urea solution coming out from the synthesis section 12, an expansion valve 16 is used.

In accordance with the invention, between the synthesis section 12 and the urea separation section 14, it is provided an expansion section 18, in a turbine 20, of the reaction mixture, essentially comprising an aqueous solution of urea and ammonium carbamate at high pressure coming from the synthesis section 12. By doing so, there is a transformation of the pressure energy of said mixture into kinetic energy on the turbine 20.

More specifically, the expansion section 18 comprises the turbine 20 which is advantageously arranged parallel to the expansion valve 16 of the prior art. Preferably, an on-off valve 22 is also provided, which is situated upstream of the turbine 20 and downstream of a branch node 24 of an outlet duct 26 of the synthesis section 12. Two portions of circuit, which respectively lead to the expansion valve 16 and to the turbine 20, extend from such branch node 24.

The reaction mixture, as stated, comprises a solution of urea and ammonium carbamate which is essentially liquid, for which reason the use of a water turbine 20, even with many stages, is suitable.

Preferably, Francis turbines are used, which are also suitable for effectively tolerating a certain percentage of vapor which is in the reaction mixture.

The turbine 20 can be associated with an electric current generator 30.

The operation of the section 18 of the invention, which is an energy production station, is clear from that which has been described and is shown hereafter.

Normally, the expansion valve 16 is closed and the on-off valve 22 is open: with this it can easily be understood how the turbine 20 works taking the high pressure aqueous solution of urea and carbamate to the medium or low pressure of the urea separation section 14.

When there is a problem with the turbine 20, or when a maintenance operation must be carried out on the turbine 20 itself, the on-off valve 22 is closed and the expansion valve 26 is opened, with the consequence of operating as the prior art.

The turbine 20 can also be placed in series with the expansion valve 16, in the case, for example, which requires high pressure reductions to be obtained: such high pressure reductions cannot advantageously be exploited with the single expansion in the turbine 20.

Regarding the operation just mentioned, i.e. in the case in which it is necessary to obtain high pressure reductions, of course, it is not excluded the possibility of placing the turbine 20 in parallel with the expansion valve 16 and of providing an adjustment valve instead of the on-off valve 22 spoken of above: in this way one normally operates with the expansion valve 16 closed and with the adjustment valve partially closed which reduces the pressure gap to be expanded in the turbine 20.

It is worth recalling the fact that the turbine 20 is realized using special materials known in the prior art, suitable for withstanding the attacks of the urea and carbamate solution which is particularly corrosive.

Of course, by associating the turbine 20 with the electric current generator 30, it is produced electrical energy which can, for example, be used for any electrical requirement of the plant 10, from the lighting of the building to the power supply for the machines that are in the plant itself 10, such as, for example, the pumps 512 and the compressors 412 of the synthesis section 12.

Furthermore, the process for urea production according to the present invention is clear from the description of the plant 10: it fundamentally consists of inserting, between a synthesis step and an urea separation step, an expansion step, in a turbine, of the aqueous solution of urea and carbamate coming from the synthesis step.

Moreover, it is evident how the present invention also concerns a method for transforming a pre-existing urea plant, equipped with a synthesis section 12 and an urea separation section 14 separated by an expansion valve 16: it is sufficient to provide an installation step of a turbine 20, which is arranged between the synthesis section 12 and urea separation section 14, preferably in parallel to the expansion valve 16.

The main advantage achieved by the process and plant for urea production with energy recovery of the present invention consists of the fact that it involves a very substantial energy saving.

Moreover, the energy is recovered in the form of kinetic energy which is particularly flexible energy: indeed, it can be easily transformed into electrical energy or it can advantageously be used for the direct actuation of apparatuses of the plant itself.

A further advantage of the process and plant for urea production with energy recovery of the present invention is that of the possibility of a particularly easy transformation of conventional plants.

As an example, it has been noted that in a plant for urea production of the type represented in figures 1-2, which produces 1500 tons of urea per day and in which the synthesis section operates at a pressure of about 140 bar, whereas the urea separation section operates at about 3 bar, in the expansion section a power of about 550 kW is advantageously obtained.

The process and plant for urea production with energy recovery of the present invention just described are susceptible to other variants and modifications, all within reach of the person skilled in the art and, as such, covered by the scope of protection of the present invention, defined by the following claims.

## Claims

1. Process for urea production, of the type comprising a high pressure synthesis step of ammonia and carbon dioxide and a medium or low pressure urea separation step, **characterized in that** it comprises an expansion step, in a turbine (20), of a reaction mixture coming from said high pressure synthesis step.

2. Process according to claim 1, **characterized in that** said turbine (20) is a water turbine and **in that** said reaction mixture comprises an aqueous solution of urea and ammonium carbamate.

3. Plant (10) for urea production with energy recovery, of the type including a high pressure synthesis section (12) of ammonia and carbon dioxide and a medium or low pressure urea separation section (14), **characterized in that** it comprises an expansion section (18) in a turbine (20) of a reaction mixture coming from said high pressure synthesis section (12).

4. Plant (10) according to claim 3, **characterized in that** said turbine (20) is a water turbine and **in that** said reaction mixture comprises an aqueous solution of urea and ammonium carbamate.

5. Plant (10) according to claim 4, **characterized in that** said water turbine (20) is of the Francis type.

6. Plant (10) according to claim 3, **characterized in that** said turbine (20) has many stages.

7. Plant (10) according to claim 3, **characterized in that** said turbine (20) is associated with an electric current generator (30).

8. Plant (10) according to claim 3, **characterized in that** said turbine (20) is realized with special materials to withstand attacks of said reaction mixture.

9. Plant (10) according to claim 3, **characterized in that** said turbine (20) is arranged in parallel to an expansion valve (16) placed between said synthesis section (12) and said urea separation section (14).

10. Plant (10) according to claim 9, **characterized in that** an on-off valve (22) is provided, which is situated upstream of said turbine (20) and downstream of a branch node (24) of an outlet duct (26) of said synthesis section (12), from said branch node (24) extending two portions of circuit, which respectively lead to said expansion valve (16) and to said turbine (20).

11. Plant (10) according to claim 3, **characterized in that** said turbine (20) is arranged in series with an expansion valve (16) which is placed between said synthesis section (12) and said urea separation section (14).

12. Plant (10) according to claim 9, **characterized in that** an adjustment valve is provided, which is situated upstream of said turbine (20) and downstream of a branch node (24) of an outlet duct (26) of said synthesis section (12), from said branch node (24) extending two portions of circuit, which respectively lead to said expansion valve (16) and to said turbine (20).

13. Method for transformation of a pre-existing urea plant, of the type comprising a high pressure synthesis section (12) of ammonia and carbon dioxide and a medium or low pressure urea separation section (14), between said sections (12, 14) an expansion valve (16) being placed, **characterized in that** it includes an installation step of a turbine (20), which is arranged between said synthesis section (12) and said urea separation section (14).

14. Method according to claim 13, **characterized in that** said turbine (20) is a water turbine and **in that** it is arranged in parallel with said expansion valve (16).
